# DEMANDE DE BREVET EUROPEEN

(11) **EP 3 903 839 A1**
(43) Date de publication de la demande: **03.11.2021**
(21) Numéro de dépôt: 20315222.8
(22) Date de dépôt: 27.04.2020
(51) Int. Cl.: A61L 9/16, A41D 13/11, B32B 1/00

(54) **MASQUE DE PROTECTION SEMI-RIGIDE D'UNE PARTIE DU VISAGE**

(71) Demandeur: Imprimerie Trulli, 06140 Vence (FR); SMCI, 98000 Monaco (MC)
(72) Inventeur: Trulli, Michel, 06140 Vence (FR); Martin, Emma, 98000 Le soleil d'Or (MC)
(74) Mandataire: Loyer & Abello

(57) **Abrégé**

L'invention concerne un masque de protection semi-rigide à usage unique ou non et destiné à protéger une partie du visage des projections ou de la transmission d'agents pathogènes d'un autre individu

Masque (1) de protection pour le visage comprenant un corps (2) et au moins un moyen d'attache (3) caractérisé en ce qu'il est semi-rigide et réalisé en une seule pièce Les moyens d'attache (3) sont le prolongement du corps (2) du masque (1) et de forme à entourer les oreilles pour reposer sur celles-ci.

Le masque selon l'invention comprend au moins 2 couches de matériau, la couche (5) une est une pellicule en plastique biodégradable et la couche (6) est en matériau semi-rigide.

L'invention se rapporte également à un procédé de fabrication dudit masque (1).

## Description

La présente invention concerne un masque de protection semi-rigide d'une partie du visage et en particulier la région du nez et de la bouche.

La présente invention se rapporte à un masque semi-rigide à usage unique ou non et destiné à protéger une partie du visage des projections ou de la transmission d'agents pathogènes d'un autre individu.

Au regard de la crise sanitaire mondiale et pour éviter la propagation d'agents pathogènes et en particulier les virus, il est recommandé de se protéger des projections de salive ou de tout autre moyen de transmission sur le visage de ces agents. Or la crise sanitaire s'accompagne d'une pénurie de moyen de protection et en particulier de masques de protection.

Le masque semi-rigide selon la présente invention, assure la protection de la bouche et du nez tout en permettant une réutilisation car il peut être nettoyé et désinfecté. Sa fabrication rapide, de faible coût et ne nécessitant pas de matériel spécifique et onéreux répond à la demande de protection en grande quantité pour couvrir tous les besoins des populations.

Il existe des dispositifs de protection rigides intégrant ou non un système de respiration ou de filtration dont un exemple est le WO2019110895, pouvant comprendre une partie supérieure destinée à protéger également les yeux. Ces dispositifs sont volumineux. Ils nécessitent des matériaux particuliers et leur coût de fabrication est onéreuse.

Il existe également des systèmes de protection souple en papier ou en fibre tissée ou non pouvant être des masques chirurgicaux comme décrit dans le brevet FR2853497. Ce brevet décrit un masque actif de protection contre la transmission d'élément pathogènes aux voies aériennes respiratoires d'un utilisateur. Ces systèmes de protection sont à usage unique avec des durées d'utilisation de quelques heures notamment des recommandations d'utilisation au maximum de 4 heures. De plus, compte tenu de la pénurie, les prix pour accéder à ces produits sont devenus trop importants pour un usage normal et ils ne sont pas recyclables.

Le masque selon l'invention permet de remédier à cet inconvénient. En effet, l'invention se rapporte à un masque (1) de protection pour le visage comprenant un corps (2) et au moins un moyen d'attache (3) caractérisé en ce qu'il est semi-rigide et réalisé en une seule pièce. Préférentiellement, le masque selon l'invention comprend 2 moyens d'attache (3) qui sont le prolongement du corps (2) du masque (1) et de forme à entourer les oreilles pour reposer sur celles-ci. Le masque selon l'invention est caractérisé en ce qu'il comprend au moins 2 couches de matériau, la couche (5) est une pellicule en plastique biodégradable et la couche (6) est en matériau semi-rigide. Le matériau semi-rigide étant choisi parmi le papier PEFC, l'offset ou le couché allant du grammage 150 à 400 gr. et de préférence du grammage 150 à 350 gr., le carton plastifié et le pvc.

Le masque selon l'invention comprend une couche optionnelle (7) de pellicule en plastique biodégradable. Dans un mode de réalisation particulier, le masque selon l'invention comprend 3 couches de matériau, les 2 couches (5) et (7) sont une pellicule en plastique biodégradable et la couche (6) est un papier PEFC. Dans un autre mode de réalisation, le masque selon l'invention comprend au moins une rainure (4) destinée à épouser la forme du nez.

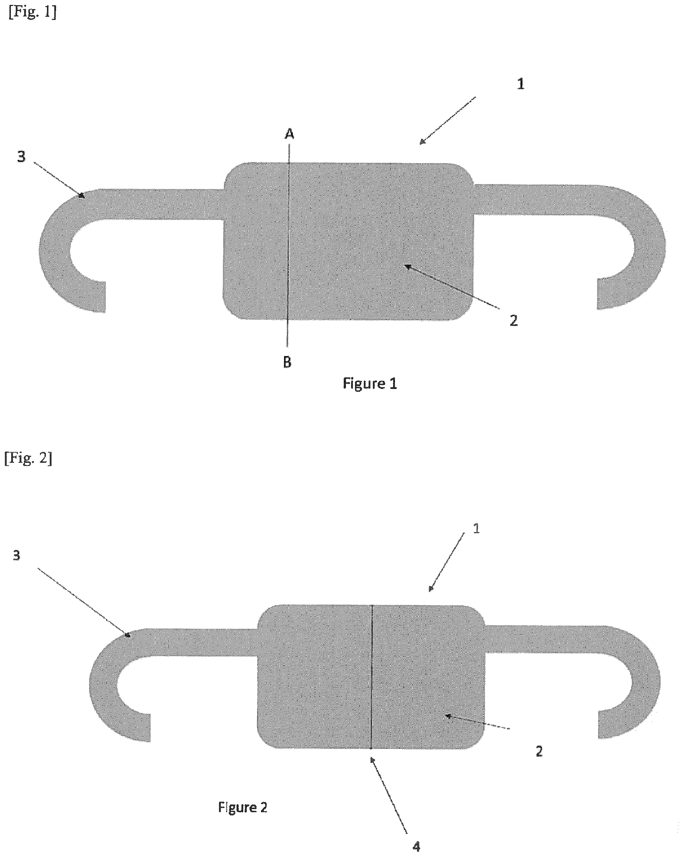

Une première caractéristique du masque (1) selon l'invention est qu'il est réalisé en une seule pièce comportant un corps (2) et des systèmes d'attache (3) comme montré en figure 1. Selon un mode de réalisation particulier, le masque comporte au moins une rainure de préférence positionnée approximativement en son centre comme indiquée en figure 2.

Le masque selon la présente invention comporte au moins 2 couches :
Une 1ere couche (4) est une pellicule de film biodégradable
Une 2èmè couche (6) de matériau semi-rigide de type papier, carton ou plastique.

Dans un mode de réalisation de l'invention, une 3^{ème} couche est également présente. Cette 3^{ème} couche (7) est pellicule protectrice de la couche (5) et peut être réalisée dans un matériau plastique biodégradable ou non. Dans un mode préféré, la 3ème couche est une pellicule de film biodégradable.

La pellicule de film biodégradable (5) selon l'invention est une pellicule transparente ou opaque d'un matériau capable de former un film d'une épaisseur d'au moins 15 µm (microns), de préférence d'une épaisseur comprise entre 20 µm et 200 µm, préférentiellement entre 20 µm et 100 µm et de manière encore plus préférée entre 25 µm et 50 µm.

La pellicule de film biodégradable est suffisamment résistante à l'eau et à l'alcool pour permettre un nettoyage avec une éponge ou une étoffe de type tissus ou coton, imbibée d'eau savonneuse ou d'alcool, notamment d'alcool à 70° ou 90° de manière à nettoyer et/ou désinfecter le masque (1).

Selon un mode particulier de l'invention, le film biodégradable est fabriqué à partir d'un plastique oxo-biodégradable et de préférence homologué pour ne pas relarguer de particules comme par exemple les plastiques oxo-biodégradable pouvant être mis en contact avec de la nourriture.

Le matériau semi-rigide constitutif de la couche (6) telle que décrite précédemment est un matériau de type papier PEFC, offset ou couché allant du grammage 150 à 400 gr. et de préférence du grammage 150 à 350 gr. ou un carton plastifié ou bien encore un pvc lui conférant de la tenue lorsqu'il est porté. Il constitue ainsi un écran protecteur de la bouche et du nez qui ne sera pas traversé comme cela peut être le cas avec des masques chirurgicaux trop fins.

Dans une mode de réalisation particulier, la couche (6) est imprimée. Dans le cadre de pandémie, la couche (6) imprimée peut indiquer les recommandations sanitaires à observer ou tout autre message.

Dans un mode de réalisation encore plus particulier, le masque selon l'invention est réalisé en plusieurs dimensions de sorte à correspondre à différentes tailles du visage pour s'adapter à la morphologie du porteur du masque. Ainsi, plusieurs tailles de masque peuvent être réalisées et de manière illustrative mais non limitative, représenter les tailles suivantes :
- Taille L : 40x 13.5 cm
- Taille M : 38 x 12.5 cm
- Taille S : 36.8 x 12.5 cm
- Taille XS : 34x 12.5 cm

L'invention se rapporte également à un procédé de fabrication du masque (1) tel que décrit précédemment, comprenant les étapes suivantes :
1. Prendre un matériau semi-rigide de type papier, carton ou plastique tel que décrit précédemment et destiné à être la couche (6),
2. Optionnellement l'imprimer à l'aide de tout moyen de reprographie sur l'un ou les deux côtés du matériau
3. Appliquer sur la couche (6) une pellicule de film biodégradable pour obtenir la couche (4)
4. Optionnellement, appliquer une pellicule de film biodégradable sur l'autre côté de la couche (6) afin de réaliser la couche (7)
5. Découper le masque (1) selon les contours définis ou imprimés selon l'étape 2, dont des exemples illustratifs sont donnés dans les figures (figures 1, 2, 4 et 5) ; la forme du masque (1) n'étant pas limitée aux produits illustrés. Cependant, la forme du masque (1) doit permettre d'obtenir celui-ci en une seule pièce et être apte à être porté pour protéger ainsi la bouche et le nez.

Les dessins annexés illustrent l'invention :
[Fig.1] représente une illustration du masque (1) selon l'invention pourvu d'un corps (2) et d'au moins un système d'attache (3).
[Fig.2] représente une illustration d'une variante (avec rainure) de la figure 1
[Fig.3] représente une coupe transversale selon [A]-[B] du masque (1)
[Fig.4] est une photo du masque (1) réalisé selon une variante de l'invention présentée dans la figure 2
[Fig. 5] est une est une photo du masque (1) réalisé selon une variante de l'invention présentée dans la figure 2

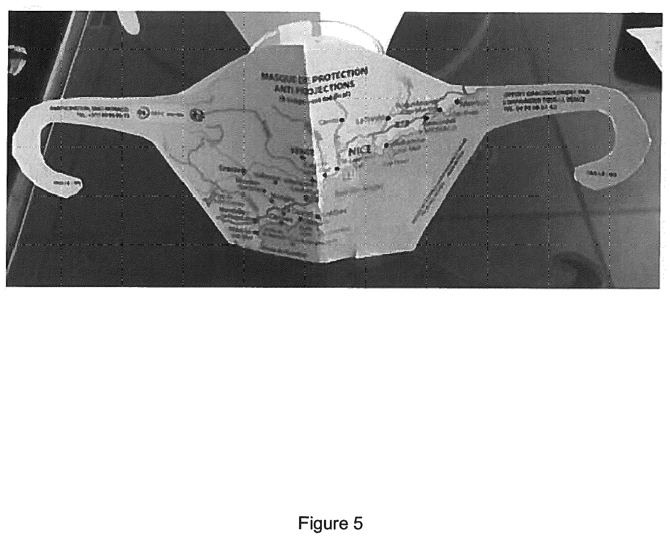

En référence à ces dessins, la figure 2 présente une variante de l'invention, dans laquelle au moins une rainure (4) a été pratiquée. Selon l'invention, la rainure est une entaille longue, de section rectangulaire, en forme de T ou en queue d'aronde, pratiquée dans le masque (1) de manière plus ou moins appuyée et/ou plus ou moins profonde. La rainure (4) pratiquée au centre du masque (1) est destinée à permettre d'accueillir le nez et ainsi permettre au masque d'épouser la forme du visage. La figure 5, illustre la variante dans laquelle la rainure est approximativement au centre du masque (1) de manière à permettre un pliage apte à former un espace destiné à accueillir le nez ou le bout du nez du porteur.

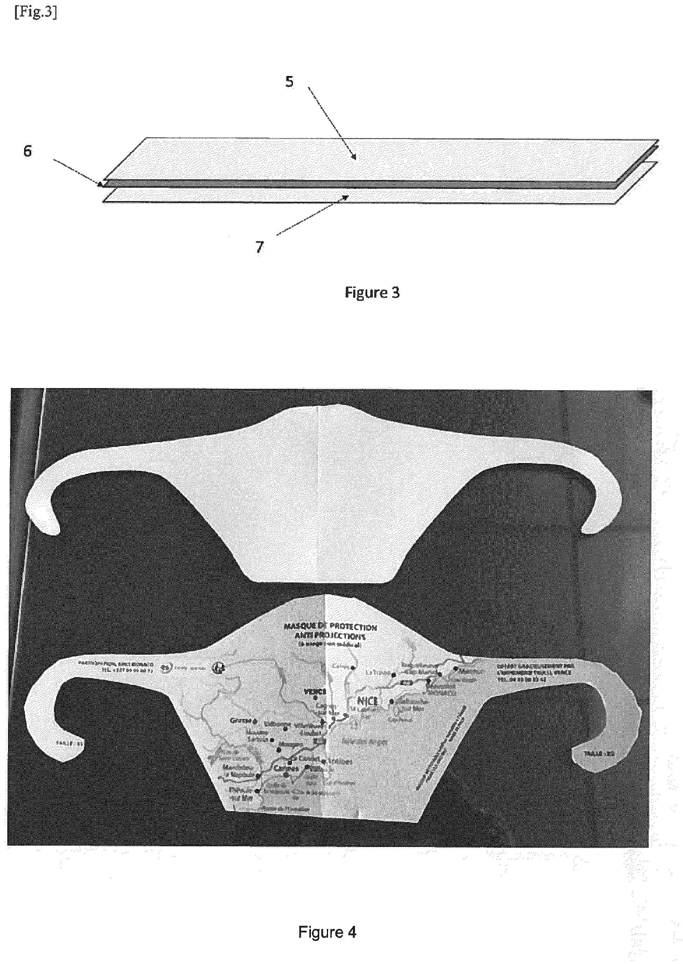

La figure 3 qui représente une coupe transversale du corps (2) du masque (1) permet de mettre en évidence les différentes parties constitutives du masque (1). Cette figure montre une variante de l'invention comportant 2 couches constitutives (5) et (6) et une 3^{ème} couche optionnelle (7). En particulier, la figure 3 décrit une couche supérieure (5) d'une pellicule de film biodégradable, une couche (6) d'un matériau semi-rigide et une couche (7) optionnelle d'une pellicule de film biodégradable.

Dans une variante de l'invention non représenté, le masque (1) comprend au moins 2 trous, de préférence de 3 à 4 mm de diamètre, proches des moyens de fixation, afin d'ajouter un moyen de fixation supplémentaire de type élastique ou ruban.

Le masque (1) selon l'invention est particulièrement destiné à la protection de la bouche et du nez d'une personne.

## Revendications

1. Masque (1) de protection pour le visage comprenant un corps (2) et au moins un moyen d'attache (3) **caractérisé en ce qu'**il est semi-rigide

2. Le masque selon la revendication 1 **caractérisé en ce qu'**il est réalisé en une seule pièce

3. Le masque selon la revendication 1 ou la revendication 2 **caractérisé en ce qu'**il comprend 2 moyens d'attache (3)

4. Le masque selon la revendication 3 **caractérisé en ce que** les moyens d'attache sont le prolongement du corps (2) du masque (1).

5. Le masque selon la revendication 4 **caractérisé en ce que** le prolongement du corps (2) des moyens d'attache (3) est de forme à entourer les oreilles pour reposer sur celles-ci.

6. Le masque selon l'une des revendications précédentes, **caractérisé en ce qu'**il comprend au moins 2 couches de matériau la couche (5) est une pellicule en plastique biodégradable et la couche (6) est en matériau semi-rigide.

7. Le masque selon la revendication 6 est **caractérisé en ce que** le matériau semi-rigide est choisi parmi le papier PEFC, l'offset ou le couché allant du grammage 150 à 400 gr.et de préférence du grammage 150 à 350 gr., le carton plastifié et le pvc

8. Le masque selon la revendication 6 ou 7, **caractérisé en ce qu'**il comprend une couche optionnelle (7) de pellicule en plastique biodégradable

9. Le masque selon l'une des revendications précédentes, **caractérisé en ce qu'**il comprend 3 couches de matériau, les 2 couches (5) et (7) sont une pellicule en plastique biodégradable et la couche (6) est un papier PEFC

10. Le masque selon l'une des revendications précédentes, **caractérisé en ce qu'**il comprend au moins une rainure (4) destinée à épouser la forme du nez

11. Procédé de fabrication d'un masque (1) selon l'une des revendications précédentes, comprenant les étapes suivantes :
1. Prendre un matériau semi-rigide de type papier, carton ou pvc selon la revendication 7 et destiné à être la couche (6),
2. Optionnellement l'imprimer à l'aide de tout moyen de reprographie sur l'un ou les deux côtés du matériau
3. Appliquer sur la couche (6) une pellicule de film biodégradable pour obtenir la couche (4)
4. Optionnellement, appliquer une pellicule de film biodégradable sur l'autre côté de la couche (6) afin de réaliser la couche (7)
5. Découper le masque (1) selon les contours définis ou imprimés selon l'étape 2.
